Europäisches Patentamt

European Patent Office   (11) Publication number:   **0 355 941**

Office européen des brevets   **A1**

(19)

(12) **EUROPEAN PATENT APPLICATION**

(21) Application number: **89301581.8**   (51) Int. Cl.4: **A01K 67/033**

(22) Date of filing: **17.02.89**

(30) Priority: **20.02.88 GB 8804001**

(43) Date of publication of application:
**28.02.90 Bulletin 90/09**

(84) Designated Contracting States:
**AT BE CH DE ES FR GB GR IT LI LU NL SE**

(71) Applicant: **Hird, Gordon Edward**
**30 Taylor Road Kings Heath**
**Birmingham B13 OPG(GB)**

(72) Inventor: **Hird, Gordon Edward**
**30 Taylor Road**
**Kings Heath Birmingham B13 0PG(GB)**
Inventor: **Goode, Michael Joseph**
**Cobblers Farm Low Hill Torton**
**Hartlebury Worcestershire, DY10 4HT(GB)**

(74) Representative: **Leach, John Nigel et al**
**FORRESTER & BOEHMERT**
**Widenmayerstrasse 4/I**
**D-8000 München 22(DE)**

(54) **Snail habitat.**

(57) "A snail habitat (10) comprising a container (11), having an internal surface (30), which is adapted to provide a habitat for snails, means to induce snails to migrate away from a part of the surface and cleaning means to direct cleaning fluid onto said part of the surface to clean said surface part."

EP 0 355 941 A1

## Snail Habitat

This invention relates to a snail habitat.

Snails are currently intensively farmed in a habitat provided by static containers. The snails may make use of all the internal surfaces of the container and deposit mucus and other excreta whenever they move.

It is therefore necessary to clean the interior of the containers to remove this excreta. Hitherto, this has been done manually, generally with a sponge and water and this has rendered snail farming a labour intensive cottage style industry. Moreover, frequent enforced movement or handling of the snails or an excessive volume of water can seriously retard growth and may cause death of at least some of the snails.

Accordingly, an object of the present invention is to provide a snail habitat whereby the above mentioned cleaning problem is overcome or is reduced.

According to one aspect of the invention we provide a snail habitat comprising a container, having an internal surface, which is adapted to provide a habitat for snails, means to induce snails to migrate away from a part of the surface and cleaning means to direct cleaning fluid onto said part of the surface to clean said surface part.

The part of the surface to be cleaned may vary.

The means to induce the snails to migrate away from said surface part may comprise illuminating means to illuminate said part with light or other radiation to which the snails react by moving away from an illuminated region.

The means to induce snails to migrate away from said part may alternatively, or in addition, comprise means to change the orientation of said part relative to gravity. Since the snails prefer a surface which is at least partly inverted changing said surface part from an inverted or more inverted orientation to a less inverted or to an erect orientation induces said snails to migrate away from said part.

According to a second aspect of the invention we provide a snail habitat comprising a container, having an internal surface, which is adapted to provide a habitat for snails, cleaning means to direct cleaning fluid in a localised flow path onto a part only of said surface, and means to cause relative movement between said flow path and said surface to permit cleansing thereof.

In both aspects:-

The container may be mounted for rotation, about an axis inclined to the vertical, and preferably also inclined to the horizontal, during a cleaning cycle, so that a first surface part which is disposed in an inverted or more inverted orientation at a first position in a first operational state of the habitat at the beginning of the cleaning cycle may be moved so as to be disposed in a less inverted or an erect orientation at a second position in a second operational state of the habitat at the end of the cleaning cycle, the cleaning means being adapted to direct said cleaning fluid onto said first surface part when the first surface part is in said second position.

Preferably the angle of inclination of the container relative to the vertical is not more than 45° and preferably not more than 30°.

Means may be provided to rotate the container through an extent necessary to ensure adequate cleaning, for example, through half, or one and a half, or any other number of complete revolutions plus a half revolution, during the cleaning cycle so that said first surface part is disposed in an erect or more erect orientation at said second position, which is diametrically opposite the first position, at the end of the cycle.

The illuminating means may be adapted to illuminate said surface part at said second position.

The container may comprise a cylindrical internal surface the opposite ends of the cylinder being provided with means to contain snails therein.

At least at one end said means may comprise a region which is provided with an environment which causes the snails not to cross said region. For example, the region may be provided with means to provide an electric shock to any snails entering the region or attempting to enter the region.

At both ends the container may be closed by a mechanical means such as a solid or foraminous end wall extending generally transversely to the axis of rotation of the container.

A feeding surface may be provided within the container and may be positioned at or adjacent either end wall.

Where the container is mounted for rotation about an axis inclined at an acute angle to the vertical said feeding surface may be at or adjacent the top end wall of the container.

The top end surface may comprise a removable light transmitting lid.

The cleaning means may comprise at least one spray nozzle disposed within the container and adapted to spray cleaning fluid onto the internal surface of the container, the or each spray nozzle being disposed so as to direct fluid onto the surface of the container at said second position which is the lowermost part of the path followed by the side wall of the container.

It has been found that the fluid discharged from a spray nozzle stimulates the snails into activity and this encourages the snails to move and feed. Thus the spray nozzle may be used for spraying more often than required strictly for cleaning the habitat.

The spray nozzle may be fed with fluid via a conduit which enters the container within a hollow bearing means by which the container is mounted for rotation.

The container may be provided with drain openings at positions which occupy the lowermost portion of the container when in said second position.

The or each spray nozzle may provide a predetermined spray pattern adapted to interfere to the least extent possible with the well-being of the snails.

A device may be provided to wipe or brush at least some of the internal surfaces of the container.

A feed means may be provided to feed food to the feed region.

The feed means may comprise a feed tube through which food may fall under gravity onto the feed region.

According to a third aspect of the invention we provide a method of cleaning a snail habitat including the step of directing cleaning fluid onto a part of the surface.

Where the habitat is mounted for rotation, the method may include the step of rotating the habitat whilst directing the cleaning fluid.

The fluid may be directed only onto said part of said surface.

Snails may be induced to migrate away from said part of the surface prior to directing said cleaning fluid thereon.

An embodiment of the invention will now be described by way of example with reference to the accompanying drawings wherein:

FIGURE 1 is a diagrammatic axial cross-sectional view through a snail habitat embodying the invention;

FIGURE 2 is a section on the line 2-2 of Figure 1, and

FIGURE 3 is a section, to a reduced scale, similar to that of Figure 2 but through a modification of the embodiment shown in Figures 1 and 2.

Referring to the drawings a snail habitat is indicated generally at 10 and comprises a container 11 having a cylindrical wall 12 closed at its lower end 13 by a base wall 14 and closable at its upper end 15 by a removable lid 16. The base wall 14 carries a boss 17 provided with a flanged bearing bush 18 within which is received a hollow axle 18 projecting from a mounting bracket 20 fixed to a support surface. The bush 18 has a pulley 21 with which a belt, not shown, is engaged to drive the pulley and hence to rotate the container 11 about the axle 19 from a geared motor, not shown. If desired the cleaning cycle can be performed automatically under suitable control such as at predetermined time intervals.

The base 14 and side wall 12 are made as a one-piece moulding in glass reinforced plastics material with the base part 14 having a central part 22 which provides a feeding area and is provided with a raised rim 23 to retain food thereon and three equally angularly spaced radially extending arms 24 which connect the side wall 12 to the central part 22 provided with the boss 17. In the regions 25 between the arms 24 is provided a plastics or metal mesh of appropriate size which permits snails to travel thereover whilst providing a surface which induces them not to remain thereon.

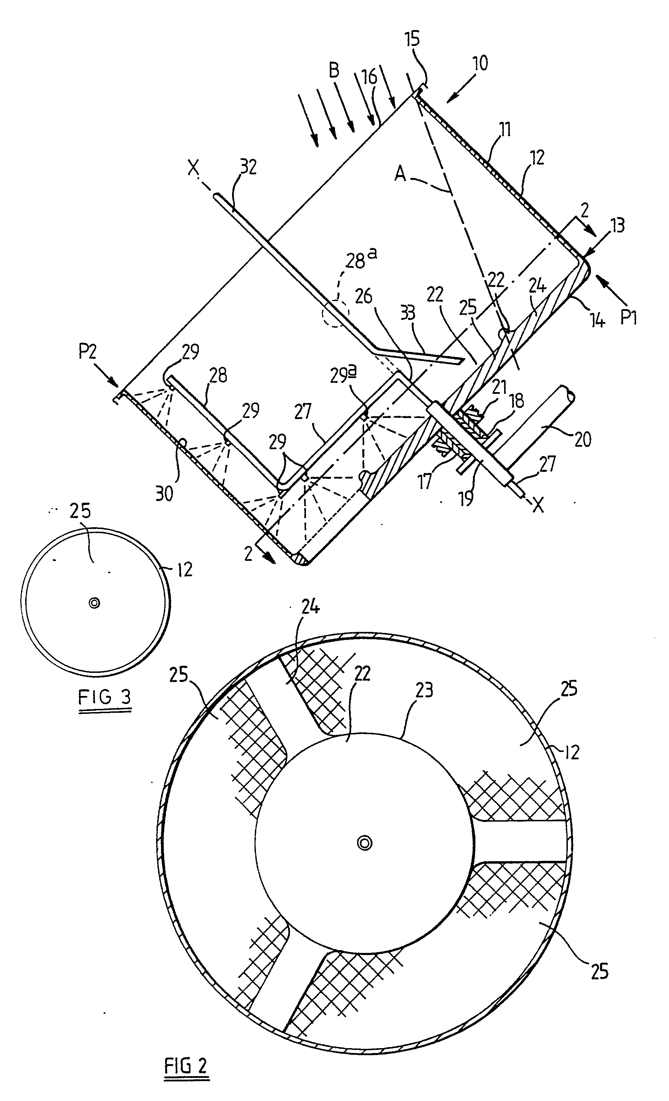

In an alternative, and preferred, embodiment the base 14 comprises a circular disc of solid plastics material which may or may not be provided with a raised rim 23 depending upon whether or not it is desired to feed the snails on the base 14. It is preferred not to feed the snails on the base 14 but instead to feed the snails by sprinkling food on the underside of the lid 16 when the lid has been removed and the feed being retained thereon by virtue of the inside surface of the lid being wet. It is preferred that the lid 16 is made of transparent plastics material so as to permit light to enter the container as shown by the arrows B. The above described alternative construction of the base 14 is shown in Figure 3.

Extending through the hollow axle 19 is a pipe 26 connected at its outer end 27 to a hose or other water supply, not shown. The pipe 26 has a transversely extending limb 27 and a vertically extending limb 28 both of which are provided with an appropriate number of spray nozzles 29 so as to spray water over the entire internal surface 30 of the container as the container is rotated past the limbs 27, 28 except for the feeding area 22 which can be cleaned manually at lesser intervals than the remainder of the container or can be cleaned by a interior spray 29a actuated less frequently than is the remainder of the container to avoid wastage of food.

A light source is disposed exteriorly of the container 11 so as to illuminate the region of the container disposed to the left of the line A in Figure 1, as indicated by the arrows B; the region to the right of the line A is in shadow. Alternatively the container may be provided with light transmitting windows which are lit to illuminate a desired region of the container. In all cases artifical or natural light may be used. Preferably the container is illuminated so as to simulate the length of a spring day i.e. about 18 hours light and 6 hours dark, since this improves the snail growth rate.

In addition, other environmental features may be provided to encourage snail growth. For example, a relatively high humidity of the order of 85% and a temperature of about 21° may be provided using appropriate heating and humidifying means for the atmosphere within which the container is located.

Where it is desired to feed the snails at the bottom of the container a feed tube 32 is disposed on the axis of rotation X-X of the container so as to extend inwardly from the top end 15 through an aperture in the lid 16 and is provided with an offset part 33 so that food can be deposited on the feeding area 22. Food may be fed down the tube 32 either manually or automatically by a timed feeding device.

In use, snails will tend to congregate on the side wall 12 when it is in an inverted condition i.e. on the part of the surface 11 occupying a first position indicated at P1 in Figure 1. The snails will tend to migrate towards a line on the surface 11 which is at the maximum inversion which line is a line parallel to the axis of rotation X-X and at the highest extent of the part of the surface 11 at the position P1. The snails tend to congregate in this region, on a first part of the surface 11 at the first position P1, because of the maximum inversion of the surface and also because the region is in -shadow being on the right hand side of the line A.

When it is desired to clean the habitat, which would typically be done once or twice a day, the container 11 is rotated about the axis X-X through 270° at a rate of approximately 5 minutes per revolution so that the container is rotatated for approximately 7 minutes. As a result the first part of the container 11 which was at the first region P1 will be rotated through a second position P2 which is centered on a line parallel to the axis and at the lowermost position occupied by the surface 11 and is then rotated back to the first position P1 and then onto the position P2 again where rotation is stopped. The snails at the original first part of the container 11 are thus now disposed at the position P2 and in light and on a surface which is not inverted. The snails find such conditions unattractive and thus tend to migrate back towards the first position P1 to congregate on the part of the surface of the container now disposed thereat. During their migration from the position P1 to the position P2 many will tend to travel over the feeding area 25 but because this is illuminated they do not tend to stay there but move on after feeding to the position P1.

However, in a preferred embodiment food is not provided on the feeding area 25. Instead, food is sprinkled on the inner surface of the lid, the lid having previously been removed, and the food being retained on the lid, because, after washing of the interior of the container the lid is wet which causes food to adhere to the lid when the lid is replaced on the container.

As a result, the snails are induced to migrate from the position P1 to the position P2 over the lid and feed on the way.

If desired the container may be rotated more than twice a day and through a different number of revolutions or part revolutions. The speed of rotation may be different to that mentioned above.

The maximum amount of excreta occurs on the first part of the surface at the region P1 because the snails stay on this region for the longest period of time. Therefore, as the snails migrate away from the original first surface part which has now disposed at the position P2 they leave behind the excreta. Thus, at the next cleaning cycle when the container is rotated and the water spray is switched on the water can clean the original first part at the position P2 without cleaning being obstructed by the presence of snails. At the beginning of a cleaning cycle the dirty first region is at the position P2 and is thus in position to be cleaned by the water sprays and is rotated through this position again during a cleaning cycle before passing on to be in the first position P1. During this period the snails which have occupied a new 'lfirst region" at the position P1 are moved to the position P2 from which they will migrate during the inter-cleansing interval to the position P1 thus leaving this further part of the surface free of snails for cleaning at the next cleaning cycle. Although it is preferred to rotate the container through a half revolution or multiples thereof, the container may be rotated through less than one half revolution or through more than one half revolution but not necessarily multiples of one half. The container may be rotated through any desired extent and may be rotated through different amounts during different cleaning cycles so long as the snails are induced to migrate adequately to expose for cleaning a part of the container surface which was previously occupied by snails.

If desired the container may be of other construction than that described hereinbefore and may be of other than cylindrical configuration. For example, it may be of polygonal shape with the length of the limb 27 chosen so as to permit of rotation of the container whilst the spray nozzles are chosen to provide a spray pattern which copes with the variation in distance between the polygonal side wall and the spray nozzles whilst achieving satisfactory cleaning without unduly disturbing the snails. Alternatively the container wall be frustoconical or of other shape and, irrespective of shape, may be provided with additional snail supporting surfaces within the container.

Furthermore, the container may be made of other material than glass reinforced plastics material such as a suitable metal such as galvanised steel or stainless steel.

The angle of inclination of the axis X-X shown is about 45° but may vary from this angle and is preferably not more than 30°. If desired the axis may be horizontal or may be vertical. Where the axis X-X is vertical then no inducement to movement of the snails can be achieved by variation in the orientation of the surface of the container and it will be necessary to reply solely on the application of light to illuminate different regions of the container to drive the snails away from a thus illuminated region so that it is available for unobstructed cleaning.

If desired, where the container is rotated about an axis inclined to the vertical so that a different orientation of the surface of the container can be achieved migration of the snails may be induced solely as a result of change in orientation without the application of light. It is preferred however that inducement to snail movement is achieved by both application of light and change in orientation as described hereinbefore.

Instead of closing the end 15 of the container with a wire mesh lid other means for containing the snails may be provided for example by providing a region adjacent the end 15 which has an environment hostile to snails for example by providing means to apply electric shocks to the snails. Similarly, the end 13 of the container similar means may be provided to maintain the snails within the container.

In an alternative container construction the base of the container is entirely closed, except for drainage apertures adjacent the junction between the side wall and the base, and a raised floor is provided within the container upon which the snails can migrate and pass to a feeding region disposed centrally of the container and spaced above the base instead of the integral base, feed region and mesh floor described hereinbefore.

If desired, in a modification, particularly where change in orientation of the container is not relied upon to induce the snails to migrate, the container may remain stationary and the spray nozzles moved within the container so as to clean the interior of the container.

In a further modification means 28a may be provided to spray the whole of the interior of the container simultaneously. In this case rotation of the container is not essential to ensure cleaning of the whole surface of the container as such but the above described means to induce the snails to move from one part of the surface to another is necessary in order to present a part of the surface previously covered by snails for cleaning.

If desired the cleaning fluid may be recycled, after suitable treatment if desired, for further cleaning operations.

The habitat may be used for snails of any age, including newly hatched snails, as well as juvenile snails and adult snails.

Preferably a plurality of habitats are mounted on a support means and are driven from a common drive by a suitable transmission. For example, 48 habitats as described hereinbefore and mounted on such a support means. If desired the means for mounting the containers on the support means for rotation may be as described hereinbefore or other suitable mounting means may be provided.

The features disclosed in the foregoing description, or the accompanying drawings, expressed in their specific forms or in terms of a means for performing the disclosed function, or a method or process for attaining the disclosed result, or a class or group of substances or compositions, as appropriate, may, separately or in any combination of such features, be utilised for realising the invention in diverse forms thereof.

## Claims

1. A snail habitat comprising a container, having an internal surface, which is adapted to provide a habitat for snails, means to induce snails to migrate away from a part of the surface and cleaning means to direct cleaning fluid onto said part of the surface to clean said surface part.

2. A snail habitat according to Claim 1 wherein the means to induce the snails to migrate away from said surface part comprises illuminating means to illuminate said part with light or other radiation to which the snails react by moving away from an illuminated region.

3. A snail habitat according to Claim 1 or Claim 2 wherein the means to induce snails to migrate away from said part comprises means to change the orientation of said part relative to gravity.

4. A snail habitat according to Claim 3 wherein said means changes said surface part from an inverted or more inverted orientation to a less inverted or to an erect orientation.

5. A snail habitat comprising a container, having an internal surface, which is adapted to provide a habitat for snails, cleaning means to direct cleaning fluid in a localised flow path onto a part only of said surface, and means to cause relative movement between said flow path and said surface to permit cleansing thereof.

6. A snail habitat according to any one of the preceding claims wherein the container is mounted for rotation, about an axis inclined to the vertical, during a cleaning cycle, so that a first surface part which is disposed in an inverted or more inverted orientation at a first position in a first operational state of the habitat at the beginning of the cleaning cycle may be moved so as to be disposed in a less inverted or an erect orientation at a second position in a second operational state of the habitat at the end of the cleaning cycle, the cleaning means being adapted to direct said cleaning fluid onto said first surface part when the first surface part is in said second position.

7. A snail habitat according to Claim 6 wherein said axis is also inclined to the horizontal.

8. A snail habitat according to Claim 7 wherein means are provided to rotate the container so that said first surface part is disposed in an erect or more erect orientation at said second position at the end of the cycle.

9. A snail habitat according to any one of Claims 6 to 8 when dependent directly or indirectly upon Claim 2 wherein the illuminating means is adapted to illuminate said surface part at said second position.

10. A snail habitat according to Claim 6 or any one of Claims 7 to 9 when dependent on Claim 6 wherein the container is mounted for rotation about an axis inclined at an acute angle to the vertical and a feeding surface is provided at or adjacent a top end wall of the container which extends generally transversely to the axis of rotation of the container.

11. A snail habitat according to Claim 6 or any one of Claims 7 to 9 when dependent directly or indirectly on Claim 6 wherein the cleaning means comprises at least one spray nozzle disposed within the container and adapted to spray cleaning fluid onto the internal surface of the container, the or each spray nozzle being disposed so as to direct fluid onto the surface of the container at said second position which is the lowermost part of the path followed by the side wall of the container.

12. A method of cleaning a snail habitat including the step of directing cleaning fluid onto a part of the surface and

a) snails are induced to migrate away from said part of the surface prior to directing said cleaning fluid thereon, and/or

b) the habitat is rotated whilst directing the cleaning fluid.

FIG 3

FIG 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| A | FR-A-2497063 (UNIVERSITE DE FRANCHE-COMPTE) --- | | A01K67/033 |
| A | FR-A-2595910 (CLERC) --- | | |
| A | US-A-3814057 (CALVERT) --- | | |
| A | DE-A-2839261 (MKT MODERNE KUCHENTECHNIK) ----- | | |
| | | | **TECHNICAL FIELDS SEARCHED (Int. Cl.4 )** |
| | | | A01K |

The present search report has been drawn up for all claims

| Place of search | Date of completion of the search | Examiner |
|---|---|---|
| THE HAGUE | 06 DECEMBER 1989 | VON ARX V.U. |